# EUROPÄISCHE PATENTSCHRIFT

(11) **EP 0 616 512 B1**
(45) Veröffentlichungstag und Bekanntmachung des Hinweises auf die Patenterteilung: **27.08.1997**
(21) Anmeldenummer: 93922921.7
(22) Anmeldetag: 08.10.1993
(51) Int. Cl.: A61B 17/56

(54) **VERANKERUNGSELEMENT**
ANCHORING ELEMENT
ELEMENT D'ANCRAGE

(30) Priorität: 09.10.1992 DE 4234118
(43) Veröffentlichungstag der Anmeldung: 28.09.1994
(60) Teilanmeldung: 97101057.4
(73) Patentinhaber: BIEDERMANN MOTECH GmbH, D-78054 Villingen-Schwenningen (DE)
(72) Erfinder: HARMS, Jürgen, D-76133 Karlsruhe (DE); SHUFFLEBARGER, Harry, L., Miami, FL 33155 (US); BIEDERMANN, Lutz, D-78048 VS-Villingen (DE)
(74) Vertreter: Prüfer, Lutz H., Dipl.-Phys.
(86) Internationale Anmeldenummer: EP9302756
(87) Internationale Veröffentlichungsnummer: WO9408527

(56) Entgegenhaltungen:
- EP-A- 0 346 521
- EP-A- 0 487 830
- WO-A-89/00028
- DE-A- 3 916 198
- FR-A- 2 659 546
- US-A- 5 129 388

## Beschreibung

Die Erfindung betrifft ein Verankerungselement nach dem Oberbegriff des Patentanspruches 1.

Ein derartiges Verankerungselement ist aus der EP-A-0 487 830 bekannt. Das das Innengewinde aufweisende Element ist als eine Hutmutter mit einer auf einer Seite geschlossenen konzentrischen Ausnehmung ausgebildet. Die Fixierung erfolgt in der Weise, daß zunächst das das Außengewinde aufweisende Fixierelement zum Fixieren der aufzunehmenden Stange eingeschraubt wird. Anschliessend soll die Hutmutter aufgeschraubt werden um zu erreichen, daß das Fixierelement im zugehörigen Gewinde verbleibt. Hier tritt das Problem auf, daß die beiden Schenkel sich beim Einschrauben des Fixierelementes nur dann nicht nach außen aufweiten, wenn die Schenkel außerordentlich massiv sind. Schon die kleinste Aufspreizung nach außen macht das Aufsetzen der Mutter unmöglich. Darüber hinaus trägt die Hutmutter nicht zur Fixierung bei.

Aufgabe der Erfindung ist es, das Verankerungselement so auszubilden, daß die Dimensionierung der Verankerung verkleinert werden kann und die Verankerung einer höheren Dauerbelastung stand hält. Das spielt insbesondere deshalb eine Rolle, weil derartige Verankerungselemente mit den damit zu verbindenden Stangen als Bestandteil von Korrekturimplantaten für die menschliche Wirbelsäule verwendet werden und deshalb kleine Dimensionen und eine unbegrenzte Belastbarkeit anzustreben sind.

Diese Aufgabe wird durch das in Anspruch 1 beschriebene Verankerungselement gelöst.

Weitere Merkmale und Zweckmäßigkeiten der Erfindung ergeben sich aus der Beschreibung von Ausführungsbeispielen anhand der Figuren.

Von den Figuren zeigen:
- Fig. 1: eine Seitenansicht einer ersten Ausführungsform des Verankerungselementes;
- Fig. 2: eine Frontansicht auf die in Fig. 1 gezeigte Vorrichtung;
- Fig. 3: einen Schnitt entlang der Linie III - III in Fig. 1;
- Fig. 4: einen Schnitt entlang der Linie IV - IV in Fig. 2;
- Fig. 5: eine Seitenansicht einer zweiten Ausführungsform;
- Fig. 6: einen Schnitt entlang der Symmetrieebene einer abgewandelten Ausführungsform in Explosionsdarstellung;
- Fig. 8: die Darstellung gemäß Fig. 7 mit fixierter gerader Stange;
- Fig. 9: dieselbe Ausführungsform mit konvex gebogener Stange; und
- Fig. 10: dieselbe Ausführungsform mit konkav gebogener Stange.

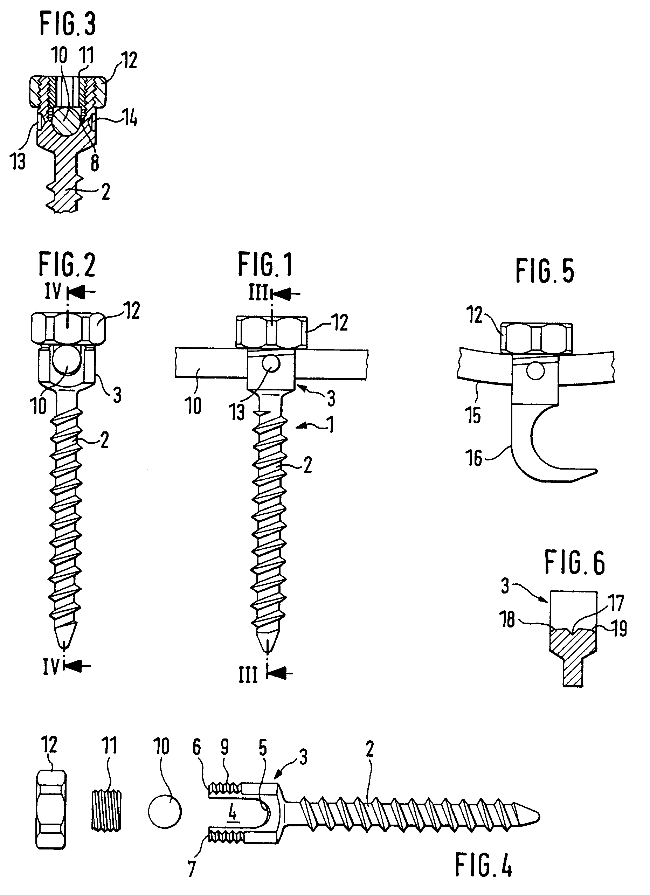

Das in Fig. 1 gezeigt Verankerungselement umfaßt eine eigentlich in eine Wirbelsäule einzubringende Schraube 1 mit einem Gewindeschaft 2 und einem Kopf 3. Der Kopf 3 weist eine zur Mittenachse des Gewindeschaftes 2 symmetrisch angeordnete U-förmige Ausnehmung 4 auf, deren Grund 5 zu dem Gewindeschaft 2 hin gerichtet ist. Die Seitenwandung des die U-förmige Ausnehmung 4 begrenzenden Kopfes wird durch freie Schenkel 6, 7 gebildet. Wie am besten aus den Fig. 3, 4, und 5 zu ersehen ist, ist im Inneren des Kanales eine konzentrisch zur Mittenachse des Gewindeschaftes 2 ausgebildete Bohrung mit einem Innengewinde 8 vorgesehen. Der Kopf 3 selbst ist zylindrisch ausgebildet und weist, wie am besten aus Fig. 4 ersichtlich ist, im Bereich der freien Schenkel 6, 7 ein Außengewinde 9 auf.

Damit mit dieser Schraube eine Stange verankert werden kann, ist ein als Gewindebolzen ausgebildetes Fixierelement 11 vorgesehen. Das Fixierelement 11 weist ein mit dem Innengewinde 8 zusammenwirkendes Außengewinde zum Einschrauben in die U-förmige Ausnehmung 4 auf. Auf seiner dem Grund 5 abgewandten Fläche weist das Fixierelement einen Schlitz oder eine entsprechend andere Ausnehmung zum Eingreifen mittels eines Schraubendrehers auf. Ferner ist ein die beiden U-förmigen Schenkel 6, 7 von außen umfassendes Element in Form einer Überwurfmutter 12 vorgesehen, deren Gewinde mit dem Außengewinde 9 zusammenwirkt. Die Drehrichtung des Innengewindes 8 und des zugehörigen Fixierelementes 11 einerseits und des Außengewindes 9 und der Überwurfmutter 12 andererseits ist entgegengesetzt gerichtet. Wie am besten aus Fig. 3 ersichtlich ist, weist der Grund 5 der U-förmigen Ausnehmung einen Radius auf, der nur soviel größer als der Radius der aufzunehmenden Stange 10 ist, daß die Stange in die U-förmige Ausnehmung leicht einsetzbar bzw. leicht aus dieser herausnehmbar ist. Das Innengewinde 8 und das Außengewinde 9 erstrecken sich jeweils so weit nach unten, also in Richtung des Grundes 5, daß die Projetion auf die Symmetrieachse einen Abstand vom Grund 5 aufweist, der kleiner ist als der Durchmesser der aufzunehmenden Stange 10.

Bei der in Fig. 5 gezeigten Ausführungsform ist der Kopf nicht mit einem Gewindeschaft, sondern mit einem als Haken 16 ausgebildeten Schaft verbunden. Dieser dient in gleicher Weise zur Verbindung mit einem Wirbelsäulenelement, wobei der Haken in die Wirbelbogen eingebracht wird.

Bei der in Fig. 6 beschriebenen Ausführungsform weist der Grund der U-förmigen Ausnehmung eine zu der Mittenachse des Innengewindes 8 konzentrische Ausnehmung 17 in Form einer Senkbohrung auf. Die beiden äußeren Ränder 18, 19 des Grundes sind nach außen zum Schaft hin abfallend gewölbt. Dadurch wird erreicht, daß beim Aufnehmen einer gebogenen Stange nicht nur eine punktförmige Auflage auf dem Grund der U-förmigen Ausnehmung 4, sondern eine größere Auflagefläche gebildet wird. Die Wölbung zum Schaft hin dient dazu, bei einer Stange, die zum Schaft hin gebogen ist, einen besseren Formschluß zu gewährleisten.

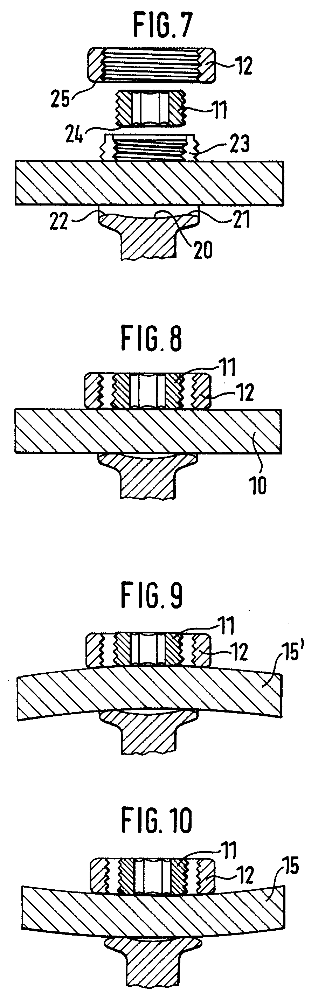

In den Fig. 7 bis 10 ist jeweils der Kopf einer weiteren Ausführungsform mit einer damit zu verbindenden Stange gezeigt. Der mit dem Kopf verbundene Schaft kann sowohl als Gewindeschaft 2 als auch als Haken 16 ausgebildet sein.

Wie aus den Figuren ersichtlich ist, ist der Grund der jeweiligen U-förmigen Ausnehmung gewölbt ausgebildet. Die Wölbung weist einen inneren konkaven Abschnitt 20 und zwei symmetrisch zur Mittenachse der Schraube angeordnete nach oben erhaben hervorstehende gewölbte Bereiche 21, 22 auf. Der Abstand der jeweils höchsten Stelle der wulstförmigen Erhebung 21 bzw. 22 von der Mittenachse der Schraube ist im wesentlichen gleich dem Abstand der Mitte 23 zwischen dem Radius des Innengewindes 8 und dem Radius des Außengewindes 9 von der Symmetrieachse der Schraube.

Wie am besten aus den Fig. 1, 3 und 5 entnehmbar ist, weist der Kopf 3 zwei gegenseitig um 180° versetzte Ausnehmungen 13, 14 auf, die im wesentlichen jeweils um 90° gegen die U-förmige Ausnehmung versetzt sind und die zum Eingreifen mittels eines Handhabungswerkzeuges, beispielsweise einer Greifzange dienen.

Im Betrieb wird zunächst die Schraube 1 in die Wirbelsäule eingeschraubt. Anschließend wird die Stange 10 bzw. 15, 15' in die U-förmige Ausnehmung 4 eingelegt und durch Einschrauben des Fixierelementes 11 in das Gewinde 8 fixiert. Dann wird die Überwurfmutter 12 aufgeschraubt. Das Fixierelement und die Überwurfmutter 12 werden nun getrennt jeweils so weit in Richtung des Grundes 5 gedreht, bis jedes der beiden Teile eine gewünschte Haltekraft auf die Stange 10, 15, 15' ausübt. Dadurch wird der Vorteil erreicht, daß die Fixierung von Fixierelement 11 und Überwurfmutter 12 relativ zu der Stange unabhängig voneinander einstellbar sind. Darüber hinaus erfolgt eine endgültige Fixation und eine Schraubensicherung durch Verklemmung.

Durch die gewölbte Ausbildung des Grundes der U-förmigen Ausnehmung und insbesondere durch die in den Fig. 7 bis 10 gezeigte Ausbildung mit den einen Abstand voneinander aufweisenden erhabenen gewölbten Auflagebereichen und deren oben beschriebene Lage wird erreicht, daß jede zu fixierende Stange, egal, ob sie als gerade Stange 10 oder als konvex oder konkav gebogene Stange 15, 15' ausgebildet ist, auf zwei flächenhaft ausgebildeten Bereichen 21, 22 aufliegt. Wie am besten aus Fig. 7 ersichtlich ist, sind zusätzlich die der Stange zugewandten unteren Ränder 24, 25 des Fixierelementes 11 und der Überwurfmutter 12 abgerundet beziehungsweise konvex gewölbt. Dadurch wird erreicht, daß alle Angriffsstellen, die bei der Fixierung auf die zu ergreifende Stange einwirken, ohne scharfe Ränder an die Stange angreifen. Darüber hinaus wird durch die beschriebene Lage erreicht, daß die Angriffsbereiche der beiden erhabenen Stellen 21, 22 des Grundes gerade zwischen den beiden Angriffsstellen des Fixierelementes 11 und der Überwurfmutter 12 liegen. Dadurch wird eine besonders gute Fixierung und eine besonders geringe Streßbelastung erreicht. Wie am besten aus den Fig. 8 bis 10 ersichtlich ist, wird die gute Fixierung für jede Art der Biegung der Stangen 10, 15, 15' erreicht. Bei den oben beschriebenen Ausführungsbeispielen ist der Kopf 3 jeweils mit dem ein Gewinde aufweisenden Schaft beziehungsweise dem hakenförmigen Schaft fest verbunden. Nach einer abgewandelten Ausführungsform sind der jeweilige Schaft und der Kopf gelenkig miteinander verbunden.

## Patentansprüche

1. Verankerungselement mit einem zur Knochenverankerung bestimmten Schaft (2, 16) und einem mit einer Stange (10, 15) verbindbaren Kopf (3) mit einem im wesentlichen U-förmigen Querschnitt, der an seinem Grund (5) mit dem Schaft (2, 16) verbunden ist und zwei einen Kanal zur Aufnahme der Stange (10, 15) bildende freie Schenkel (6, 7) hat,
wobei die Schenkel (6, 7) ein Innengewinde (8) und ein Außengewinde (9) aufweisen,
und mit einem die Schenkel (6, 7) von außen umfassenden, ein mit dem Außengewinde zusammenwirkendes Innengewinde aufweisenden Element (12) und einem ein mit dem Innengewinde (8) der Schenkel zusammenwirkendes Gewinde aufweisendes Fixierelement (11),
dadurch gekennzeichnet, daß der Grund (5) der U-förmigen Ausnehmung (4) in seiner Richtung senkrecht zum U-förmingen Querschnitt eine gewölbten Abschnitt aufweist und daß das Element (12) als beidseitig offene Mutter ausgebildet ist.

2. Verankerungselement nach Anspruch 1,
dadurch gekennzeichnet, daß die Ränder (18, 19) des Grundes (5) nach außen zum Schaft (2, 16) hin abfallend ausgebildet sind.

3. Verankerungselement nach Anspruch 1 oder 2,
dadurch gekennzeichnet, daß koaxial zum ersten Innengewinde (8) im Grund (5) des Kanales eine Ausnehmung (17) vorgesehen ist.

4. Verankerungselement nach einem der Ansprüche 1 bis 3,
dadurch gekennzeichnet, daß die Wölbung einen zur Mitte hin vertieften Abschnitt aufweist.

5. Verankerungselement nach Anspruch 4,
dadurch gekennzeichnet, daß die Wölbung jeweils in einem Abstand von der Mitte einen hervorstehend gewölbten Abschnitt (21, 22) aufweist.

6. Verankerungselement nach Anspruch 4,
dadurch gekennzeichnet, daß der Abstand eines gewölbten Abschnittes (21, 22) im wesentlichen gleich der Mitte (23) zwischen dem Radius des Innengewindes (8) und des Außengewindes (11) ist.

## Claims

1. Anchoring member with a shaft (2, 16) to be fastened to a bone and a head (3) for connection with a rod (10, 15), the head having a substantially U-shaped cross-section which has its base (5) connected to the shaft (2, 16) and comprises two free legs (6, 7) defining a channel for receiving the rod (10, 15)
wherein the legs (6, 7) comprise an internal thread (8) and an external thread (9),
and with a member (12) embracing the legs (6, 7) at their outer side and having an internal thread cooperating with the external thread, and with a fastening member (11) having a thread cooperation with the internal thread (8) of the legs,
characterized in that the base (5) of the U-shaped recess (4) has a curved portion in a direction perpendicular to the U-shaped cross-section and that the member (12) is formed as a nut having two open rides.

2. Anchoring member according to claim 1,
characterized in that the rims (18, 19) of the base (5) are formed to slope outwardly downwards towards the shaft (2, 16).

3. Anchoring member according to claims 1 or 2,
characterized in that a recess (17) is provided in the base (5) of the channel coaxial to the first internal thread (8).

4. Anchoring member according to any of the claims 1 to 3,
characterized in that the curvature comprises a depressed portion towards the center.

5. Anchoring member according to claim 4,
characterized in that the curvature comprises a respective projecting cambered portion (21, 22) spaced from the center.

6. Anchoring member according to claim 4,
characterized in that the spacing of a cambered portion (21, 22) substantially corresponds to the center (23) between the radius of the internal thread (8) and that of the external thread (11).

## Revendications

1. Elément d'ancrage comprenant une broche (2,16), destinée à être ancrée à un os, ainsi qu'une tête (3) susceptible d'être reliée à une barre (10, 15) et qui présente une section transversale sensiblement en forme de U, dont la base (5) est reliée à la broche (2, 16), et qui est munie de deux branches libres (6, 7) formant un canal de réception de la barre (10, 15), les branches (6, 7) étant munies d'un filetage intérieur (8) et d'un filetage extérieur (9), l'élément comprenant un organe (12) entourant les branches (6, 7) de l'extérieur et présentant un filetage intérieur coopérant avec le filetage extérieur, ainsi qu'un organe de fixation (11) présentant un filetage coopérant avec le filetage intérieur (8) des branches, caractérisé en ce que la base (5) de la cavité (4) en forme de U comporte, dans sa direction perpendiculaire à la section transversale en forme de U un tronçon bombé et en ce que l'organe (12) est réalisé sous la forme d'un écrou ouvert des deux côtés.

2. Elément d'ancrage selon la revendication 1, caractérisé en ce que les bords (18,19) de la base (5) sont réalisés tombant vers l'extérieur, du côté de la broche (2, 16).

3. Elément d'ancrage selon la revendication 1 ou 2, caractérisé en ce qu'il comporte, coaxialement au premier filetage intérieur (8) dans la base (5) du canal, une cavité (17).

4. Elément d'ancrage selon l'une des revendications 1 0 3, caractérisé en ce que le bombement comporte un tronçon creusé au milieu.

5. Elément d'ancrage selon la revendication 4, caractérisé en ce que le bombement comporte, de chaque côté à distance du milieu, un tronçon (21, 22) bombé vers l'extérieur.

6. Elément d'ancrage selon la revendication 4, caractérisé en ce que la distance de positionnement d'un tronçon bombé (21, 22) est située sensiblement au niveau du milieu (23) entre le rayon du filetage intérieur (8) et le rayon du filetage extérieur (11).
